# EUROPÄISCHE PATENTSCHRIFT

(11) **EP 1 487 421 B1**
(45) Veröffentlichungstag und Bekanntmachung des Hinweises auf die Patenterteilung: **01.02.2006**
(21) Anmeldenummer: 03708180.9
(22) Anmeldetag: 06.03.2003
(51) Int. Cl.: A61K 9/50, A61K 31/58

(54) **PHARMAZEUTISCHE FORMULIERUNG FÜR DEN WIRKSTOFF BUDESONID**
PHARMACEUTICAL FORMULATION FOR THE ACTIVE INGREDIENT BUDESONIDE
FORMULATION PHARMACEUTIQUE POUR LE PRINCIPE ACTIF BUDESONIDE

(30) Priorität: 27.03.2002 DE 10214002
(43) Veröffentlichungstag der Anmeldung: 22.12.2004
(73) Patentinhaber: Röhm GmbH & Co. KG, 64293 Darmstadt (DE)
(72) Erfinder: BECKERT, Thomas, 88447 Warthausen (DE); RUDOLPH, Markus, 63263 Neu-Isenburg (DE); DRESSMAN, Jennifer, 60322 Frankfurt/Main (DE)
(86) Internationale Anmeldenummer: PCT/EP2003/002269
(87) Internationale Veröffentlichungsnummer: WO 2003/080032

(56) Entgegenhaltungen:
- WO-A-01/68058
- WO-A-95/08323
- WO-A-02/060415
- US-A- 5 643 602

## Beschreibung

### Gebiet der Erfindung

Die Erfindung betrifft eine pharmazeutische Formulierung für den Wirkstoff Budesonid

### Stand der Technik

Löfberg, R. beschreibt in "Research and Clinical Forums, Vol. 15 (5), S. 92 - 96 (1993), Budesonid Formulierungen zur oralen Therapie der "Inflammatory Bowel Desase (IBD)". Darin sind Budesonid Pellets, bestehend aus einem Zuckerkern mit einer dünnen Budesonid Schicht in einem nicht näher bestimmten, wasserunlöslichem "rate limiting polymer" und einem Überzug aus EUDRAGIT® L 100-55, beschrieben. Die Pellets können in Gelatinekapseln verfüllt werden, die die eigentliche Arzneiform darstellen.

WO 95/08323 beschreibt Budesonid-Pellets mit kontrolliertem Freigabeprofil und ein Verfahren ihrer Herstellung. Zur Verbesserung der Löslichkeit von Budesonid erfolgt der Auftrag des Wirkstoffs auf die Pelletkerne in einem Hilfsstoffgemisch. Dazu suspendiert man den Wirkstoff in einem Alkohol:Wasser Gemisch von 0:100 bis 20:80 und fügt auf einem Teil des Gemischs mindestens zwei Teile eines geeigneten wasserlöslichen Hilfsstoffs, z. B. α-Lactose-Monohydrat, Saccharose oder Mononatriumcitrat, hinzu. Um ein geeignetes Freigabeprofil zu erhalten, werden die Budesonid Kerne mit einem zweischichtigen Überzug aus z. B. EUDRAGIT® L, S, RS und/oder RL innen und EUDRAGIT® RS/RL außen überzogen.

WO 97/00512 und US 5,849,327 beschreiben Arzneiformen zur Freisetzung von Wirkstoffen wie z. B. Budesonid im unteren Gastrointestinaltrakt. Die Arzneiform enthält den Wirkstoff gebunden in vernetzten Polymerpartikel, die zusätzlich mit EUDRAGIT® 100 S (Copolymer aus Methylmethacrylat und Methacrylsäure) einem mikrobiell abbaubaren Polysaccharid überzogen werden. Die Partikel werden in Kapsel gefüllt die z. B. wiederum mit EUDRAGIT® 100 S überzogen sein können.

WO 01/68058 betrifft die Verwendung einer mehrschichtigen Arzneiform, die im wesentlichen aufgebaut ist aus a) einem Kern mit einem pharmazeutischen Wirkstoff, der z. B. Budesonid sein kann b) einem inneren Überzug aus einem Copolymeren oder einer Mischung von Copolymeren, die sich aus 85 bis 98 Gew.-% radikalisch polymerisierten C1- bis C4-Alkylestern der Acryl- oder der Methacrylsäure und 15 bis 2 Gew.-% (Meth)acrylat-Monomeren mit einer quarternären Ammoniumgruppe im Alkylrest zusammensetzen und c) einem äußeren Überzug aus einem Copolymeren, das sich aus 75 bis 95 Gew.-% radikalisch polymerisierten C₁- bis C₄-Alkylestern der Acryl- oder der Methacrylsäure und 5 bis 25 Gew.-% (Meth)acrylat-Monomeren mit einer anionischen Gruppe im Alkylrest zusammensetzt, zur Herstellung einer Arzneiform, welche im Freisetzungstest nach USP zwei Stunden bei pH 1,2 und anschließendem Umpuffern auf pH 7,0 den enthaltenen Wirkstoff im Zeitraum bis 2,0 Stunden nach Testbeginn zu weniger als 5 % und zum Zeitpunkt acht Stunden nach Testbeginn zu 30 bis 80% freisetzt. Der äußere Überzug kann von Typ EUDRAGIT® FS sein.

### Aufgabe und Lösung

Ein Problem bei pharmazeutischen Formulierungen, die den Wirkstoff Budesonid enthalten, ist die geringe Löslichkeit des Wirkstoffs. Ein Weg zur Verbesserung der Löslichkeit ist gemäß WO 95/08323 die Formulierung von Budesonid mit wasserlöslichen Hilfsstoffen.

Dazu ist es erforderlich, Budesonid in einem Alkohol:Wasser Gemisch von 0:100 bis 20:80 zu suspendieren. Dies wird als nachteilig angesehen, da man aufgrund von Umwelt- und Arbeitssicherheitserwägung heutzutage die Verwendung organischer Lösemittel stets zu vermeiden versucht.

Weiterhin muß die Formulierung mit wasserlöslichen Hilfsstoffen, z. B. α-Lactose-Monohydrat, Saccharose oder Mononatriumcitrat, erfolgen, die zu unerwünschten Nebenwirkungen führen können. Bekannt ist z. B. eine Lactose-Unverträglichkeit bei Patienten, die unter Dramerkrankungen wie Colitis Ulcerosa leiden.

Es wurde als eine Aufgabe gesehen, eine Budesonid Formulierung bereitzustellen, die Nachteile des Standes der Technik vermeidet. Die Herstellung soll ganz ohne die Verwendung organischer Lösemittel möglich sein. Hilfsstoffe zur Erhöhung der Löslichkeit wie sie in der WO 95/08323 genannt sind, sollten weitgehend vermieden werden, um das Risiko von Unverträglichkeiten zu vermindern.

Die Aufgabe wird gelöst durch eine
Pharmazeutische Formulierung, enthaltend im wesentlichen
a) eine innere Schicht, die gegebenenfalls auf einen Kern aufgetragen sein kann, mit dem Wirkstoff Budesonid, gebunden in einem Bindemittel
b) eine mittlere Schicht mit einem darmsaftlöslichen oder retardierenden, polymeren Überzugsmittel,
c) eine äußere magensaftresistente Umhüllung oder eine äußere Schicht mit einem magensaftresistenten Überzugsmittel

wobei die Schichten in an sich bekannter Weise weitere pharmazeutisch übliche Hilfsstoffe enthalten können,
dadurch gekennzeichnet, daß
das Bindemittel ein Polymer oder Copolymer mit sauren Gruppen ist und die Formulierung der inneren Schicht ohne mittlere und äußere Schicht den gebundenen Wirkstoff im Freisetzungsversuch nach USP XXIII Monographie <711 > "Dissolution" mit Apparat 2 (Paddle) bei einer Drehzahl von 100 / min in Phosphatpuffer pH 7,5 nach Monographie "Intestinal Fluid, Simulated, TS" ohne Zusatz von Pepsin nach 30 min zu mehr als 80 % freisetzt.

### Ausführung der Erfindung

Die erfindungsgemäße pharmazeutische Formulierung, enthält im wesentlichen
a) eine innere Schicht, die gegebenenfalls auf einen Kern aufgetragen sein kann, mit dem Wirkstoff Budesonid, gebunden in einem Bindemittel
b) eine mittlere Schicht mit einem darmsaftlöslichen oder retardierenden, polymeren Überzugsmittel,
c) eine äußere magensaftresistente Umhüllung oder eine äußere Schicht mit einem magensaftresistenten Überzugsmittel

wobei die Schichten in an sich bekannter Weise weitere pharmazeutisch übliche Hilfsstoffe enthalten können,
dadurch gekennzeichnet, daß
das Bindemittel ein Polymer oder Copolymer mit sauren Gruppen ist und die Formulierung der inneren Schicht ohne mittlere und äußere Schicht den gebundenen Wirkstoff im Freisetzungsversuch nach USP XXIII Monographie <711> "Dissolution" mit Apparat 2 (Paddle) bei einer Drehzahl von 100 / min in Phosphatpuffer pH 7,5 (nach Monographie "Intestinal Fluid, Simulated, TS" ohne Zusatz von Pepsin) nach 30 min zu mehr als 80 % freisetzt.

### Innere Schicht a)

Die innere Schicht, die gegebenenfalls auf einen Kern aufgetragen sein kann, enthält den Wirkstoff Budesonid, gebunden in einem polymeren Bindemittel mit sauren Gruppen.

Der Wirkstoff Budesonid wird bevorzugt in handelsüblicher mikronisierter Form eingesetzt. Die mittlere Teilchengröße kann z. B. im Bereich von 2 bis 50 µm, bevorzugt 5 bis 25 µm, insbesondere 8 bis 15 µm betragen.

Der Wirkstoff Budesonid liegt gebunden in einem polymeren Bindemittel mit sauren Gruppen vor. Die Einbindung des Wirkstoffs im polymeren Bindemittel soll bevorzugt ohne die Verwendung von organischen Lösemitteln erfolgen.

Das polymeren Bindemittel mit sauren Gruppen kann z. B. ein wasserlösliches Polymer sein, das in Form einer Dispersion zusammen mit den Wirkstoff und gegebenenfalls weiteren Hilfsstoffen z. B. durch Sprühauftrag appliziert werden kann. Auf diese Weise kann man z. B. Pellets mit einem wirkstoffhaltigen Budesonid-Überzug versehen.

Das polymere Bindemittel mit sauren Gruppen kann z. B. auch ein thermisch plastifizierbares Polymer sein, das in Gegenwart des Wirkstoffs und gegebenenfalls weiterer Hilfsstoffen aufgeschmolzen wird oder in dessen Schmelze der Wirkstoff und gegebenenfalls die weiteren Hilfsstoffe gegeben werden. Man kann z. B. wirkstoffhaltige Folien erzeugen und Kerne darin einschweißen oder die Formulierung der Schicht a) durch Sprühauftrag im Schmelzezustand aufbringen.

Die Verarbeitung kann in diesem Fall z. B. durch Spritzguß oder Extrusion erfolgen. Die Mischung kann man z. B. durch Heißabschlag in Granulatform überführen.

### Polymeres Bindemittel mit sauren Gruppen

In Sinne der Erfindung ist jedes pharmazeutisch anwendbare polymere Bindemittel mit sauren Gruppen geeignet, welches in Kombination mit dem gebundenen Wirkstoff dazu führt, daß im Freisetzungsversuch nach USP XXIII Monographie <711> "Dissolution" mit Apparat 2 (Paddle) bei einer Drehzahl von 100 / min in Phosphatpuffer pH 7,5 nach Monographie "Intestinal Fluid, Simulated, TS" ohne Zusatz von Pepsin nach 30 min mehr als 80 % des gebundenen Budesonids freisetzt werden. Dies ist nur möglich wenn polymere Bindemittel mit sauren Gruppen und das Budesonid eine Interaktion eingehen, die die Löslichkeit des Budesonids erhöhen. Der genau molekulare Mechanismus der Löslichkeitserhöhung ist dabei unbekannt. Es wird lediglich angenommen, daß die sauren Gruppen eine Rolle dabei spielen.

Geeignet sind insbesondere
polymere Bindemittel die (Meth)acrylat-Copolymere sind, welche 40 bis 95 Gew.-% radikalisch polymerisierte Einheiten von C₁- bis C₄-Alkylestern der Acryl- oder der Methacrylsäure und 5 bis 60 Gew.-% (Meth)acrylat-Monomeren mit einer anionischen Gruppe im Alkylrest enthalten. In der Regel addieren sich die genannten Anteile zu 100 Gew.-%. Es können jedoch zusätzlich, ohne daß dies zu einer Beeinträchtigung oder Veränderung der wesentlichen Eigenschaften führt, geringe Mengen im Bereich von 0 bis 10, z. B. 1 bis 5 Gew.-% weiterer vinylisch copolymerisierbarer Monomere, wie z. B. Methylmethacrylat, Butylmethacrylat, Butylacrylat oder Hydroxyethylmethacrylat enthalten sein.

C₁- bis C₄-Alkylestern der Acryl- oder Methacrylsäure sind insbesondere Methylmethacrylat, Ethylmethacrylat, Butylmethacrylat, Methylacrylat, Ethylacrylat und Butylacrylat.

Ein (Meth)acrylat-Monomer mit einer anionischen Gruppe im Alkylrest kann z. B. Acrylsäure, bevorzugt jedoch Methacrylsäure sein. Die Carboxylgruppen können bis zu 30 mol-%, bevorzugt bis zu 5 bis 15 mol-% teilneutralisiert sein.

Geeignet sind anionische (Meth)acrylat Copolymere aus 40 bis 60, Gew.-% Methacrylsäure und 60 bis 40 Gew.-% Methylmethacrylat oder 60 bis 40 Gew.-% Ethylacrylat (Typen EUDRAGIT® L oder EUDRAGIT® L 100-55).

Ebenso geeignet sind anionische (Meth)acrylat Copolymere aus 20 bis 40 Gew.-% Methacrylsäure und 80 bis 60 Gew.-% Methylmethacrylat (Typ EUDRAGIT® S).

Geeignet sind ebenfalls anionische (Meth)acrylat Copolymere aus 20 bis 34 Gew.-% Methacrylsäure und/oder Acrylsäure, 20 bis 69 Gew.-% Methylacrylat und 0 bis 40 Gew.-% Ethylacrylat und gegebenenfalls 0 bis 10 Gew.-% weiteren vinylisch copolymerisierbarern Monomeren, mit der Maßgabe, daß die Glastemperatur des Copolymers nach ISO 11357-2, Punkt 3.3.3, höchstens 60 °C beträgt. (Typ EUDRAGIT® mit mittlerem Gehalt an Methacrylsäure).

Das Copolymer setzt sich insbesondere zusammen aus radikalisch polymerisierten Einheiten von
20 bis 34, bevorzugt 25 bis 33, besonders bevorzugt 28 bis 32 Gew.-% Methacrylsäure oder Acrylsäure, bevorzugt ist Methacrylsäure,
20 bis 69, bevorzugt 35 bis 65, besonders bevorzugt 35 bis 55 Gew.-% Methylacrylat und gegebenenfalls
0 bis 40, bevorzugt 5 bis 35, besonders bevorzugt 15 bis 35 Gew.-% Ethylacrylat zusammen, mit der Maßgabe, daß die Glastemperatur des Copolymers (ohne Weichmacherzusatz) nach ISO 11357-2, Punkt 3.3.3, höchstens 60, bevorzugt 40 bis 60, besonders bevorzugt 45 bis 55 °C beträgt.

Das (Meth)acrylat-Copolymer besteht bevorzugt in wesentlichen bis ausschließlich aus den Monomeren Methacrylsäure, Methylacrylat und Ethylacrylat in den oben angegebenen Mengenanteilen. In der Regel addieren sich die genannten Anteile zu 100 Gew.-%. Es können jedoch zusätzlich, ohne daß dies zu einer Beeinträchtigung oder Veränderung der wesentlichen Eigenschaften führt, geringe Mengen im Bereich von 0 bis 10, z. B. 1 bis 5 Gew.-% weiterer vinylisch copolymerisierbarer Monomere, wie z. B. Methylmethacrylat, Butylmethacrylat, Butylacrylat oder Hydroxyethylmethacrylat enthalten sein.

Die genannten Copolymere können in an sich bekannter Weise durch radikalische Substanz-, Lösungs-, Perl- oder Emulsionspolymerisation erhalten werden. Sie müssen vor der Verarbeitung durch geeignete Mahl-, Trocken- oder Sprühprozesse in den erfindungsgemäßen Teilchengrößenbereich gebracht werden.
Dies kann durch einfaches Brechen extudierter und abgekühlter Granulatstränge oder Heißabschlag erfolgen.

Das (Meth)acrylat-Copolymere liegt bevorzugt in Form einer Dispersion z. B. mit einem Wassergehalt von 60 bis 80 Gew.-% vor. Die Carboxylgruppen können bis zu 30-mol%, bevorzugt zu 5 bis 15-mol% durch eine Base, z. B. NaOH, teilneutralisiert sein.

Die Erzeugung der inneren Schicht a) erfolgt bevorzugt durch wäßriges Sprühen einer Budesonid-haltigen (Meth)acrylat-Copolymer-Dispersion auf Kerne, z. B. Saccharose Pellets, unter Bindung des Budesonids nach dem Abdampfen bzw. der Verflüchtigung des Wassers. Die Produkttemperatur während des Sprühauftrages kann dabei z. B. 20 bis 40, bevorzugt 25 bis 35 °C betragen. In der Regel setzt man der Budesonid-haltigen (Meth)acrylat-Copolymer-Dispersion ein Trennmittel, z. B. Talkum, und einen Weichmacher, z. b. Triethylcitrat, zu. Die Verarbeitung des Budesonids und gegebenenfalls der Zusatzstoff kann bevorzugt durch Einrühren in Wasser unter zunächst heftiger Durchmischung, z. B. durch 5 bis 15 minütiges Mischen z. B. mit einem Hochgeschwindigkeitsmixer (Homogenisator) erfolgen. Die so erhaltene Suspension kann dann der (Meth)acrylat-Copolymer-Dispersion zugegeben werden. Die Mischung sollte zweckmäßigerweise und bevorzugt auch während des Sprühvorgangs kontinuierlich gerührt werden.

Geeignet sind auch
polymere Bindemittel die Vinylpyrrolidon-Vinylacetat-Copolymere sind. Der molare Vinylacetat-Anteil liegt dabei bevorzugt in einem Bereich 10 bis 60 mol-%, besonders bevorzugt bei 30 bis 50 mol-% (Geeignete Handelsprodukte sind z. B. Kollidon® VA64, BASF, Ludwigshafen, Deutschland).

Allerdings müssen die Vinylpyrrolidon-Vinylacetat-Copolymere in der Regel in Form einem Lösemittel, z. B. Ethanol, gelöst verarbeitet werden, was weniger bevorzugt ist.

Die Erzeugung der inneren Schicht a) kann in diesem Fall durch Sprühen einer Budesonid-haltigen Vinylpyrrolidon-Vinylacetat-Copolymere-Lösung, z. B. in Ethanol, auf Kerne, z. B. Saccharose Pellets, unter Bindung des Budesonids nach dem Abdampfen des Lösemittels erfolgen. Die Sprühtemperatur kann dabei z. B. 30 bis 60 °C betragen. In der Regel setzt man Budesonid-haltigen Vinylpyrrolidon-Vinylacetat-Copolymere-Lösung ein Trennmittel, z. B. Talkum, und einen Weichmacher, z. b. Triethylcitrat, zu.

### Mittlere Schicht b)

Die mittlere Schicht besteht im wesentlichen aus einem darmsaftlöslichen oder retardierenden, polymeren Überzugsmittel.

### Darmsaftlösliche polymere Überzugsmittel

Geeignet sind z. B. (Meth)acrylat-Copolymere, welches 40 bis 100 Gew.-% radikalisch polymerisierte Einheiten von C₁- bis C₄-Alkylestern der Acryl- oder der Methacrylsäure und kein oder bis zu 60 Gew.-% (Meth)acrylat-Monomeren mit einer anionischen Gruppe im Alkylrest enthalten.

Diese können identisch mit den oben für die innere Schicht a) genannten (Meth)acrylat Copolymeren sein. Bevorzugt sind die (Meth)acrylat-Copolymere verschieden vom (Meth)acrylat-Copolymer der inneren Schicht.

Geeignet sind weiterhin auch z. B. (Meth)acrylat Copolymere, bestehend aus 10 bis 30 Gew.-%, Methylmethacrylat, 50 bis 70 Gew.-% Methylacrylat und 5 bis 15 Gew.-% Methacrylsäure (Typ EUDRAGIT® FS).

### Retardierende polymere Überzugsmittel

Bevorzugt werden für die mittlere Schicht retardierende polymere Überzugsmittel verwendet.

Geeignet sind z. B. (Meth)acrylat-Copolymere, welche 85 bis 98 Gew.-% radikalisch polymerisierten Einheiten von C1- bis C4-Alkylester der Acryl- oder der Methacrylsäure und 15 bis 2 Gew.-% (Meth)acrylat-Monomeren mit einer quaternären Ammoniumgruppe im Alkylrest enthalten.

Entsprechende (Meth)acrylat-Copolymere sind z. B. aus EP-A 181 515 oder aus DE-PS 1 617 751 bekannt. Es handelt sich um unabhängig vom pH-Wert lösliche oder quellbare Polymerisate, die für Arzneimittelüberzügen geeignet sind. Als mögliches Herstellungsverfahren ist die Substanzpolymeriation in Gegenwart eines im Monomerengemisch gelösten radikalbildenden Initiators zu nennen. Ebenso kann das Polymerisat auch mittels Lösungs- oder Fällungspolymerisation hergestellt werden. Das Polymerisat kann auf diese Weise in Form eines feinen Pulvers erhalten werden, was bei der Subtanzpolymerisation durch Mahlen, bei Lösungs- und Fällungspolymerisation z. B. durch Sprühtrocknung erreichbar ist.

Das (Meth)acrylat-Copolymer, setzt sich aus 85 bis 98 Gew.-% radikalisch polymerisierten C1- bis C4-Alkylestern der Acryl- oder der Methacrylsäure und 15 bis 2 Gew.-% (Meth)acrylat-Monomeren mit einer quaternären Ammoniumgruppe im Alkylrest zusammen.

Bevorzugte C1- bis C4-Alkylestern der Acryl- oder der Methacrylsäure sind Methylacrylat, Ethylacrylat, Butylacrylat, Butylmethacrylat und Methylmethacrylat.

Als (Meth)acrylat Monomer mit quaternären Ammoniumgruppen wird 2-Trimethylammoniumethylmethacrylat-Chlorid besonders bevorzugt.

Ein entsprechendes Copolymer, kann z. B. aus 50 - 70 Gew.-% Methylmethacrylat, 20 - 40 Gew.-% Ethylacrylat und 7 - 2 Gew.-% 2-Trimethylammoniumethylmethacrylat-Chlorid aufgebaut sein.

Ein konkret geeignetes Copolymer enthält 65 Gew.-% Methylmethacrylat, 30 Gew.-% Ethylacrylat und 5 Gew.-% 2-Trimethylammoniumethylmethacrylat-Chlorid aufgebaut sein (EUDRAGIT® RS).

Ein weiteres geeignetes (Meth)acrylat-Copolymer kann z. B. aus 85 bis weniger als 93 Gew.-% C1- bis C4-Alkylestern der Acryl- oder der Methacrylsäure und mehr als 7 bis 15 Gew.-% (Meth)acrylat Monomeren mit einer quaternären Ammoniumgruppe im Alkylrest aufgebaut sein. Derartige (Meth)acrylatMonomere sind handelsüblich und werden seit langem für retardierende Überzüge verwendet.

Ein konkret geeignetes Copolymer enthält z. B. 60 Gew.-% Methylmethacrylat, 30 Gew.-% Ethylacrylat und 10 Gew.-%
2-Trimethylammoniumethlymethacrylat-Chlorid (EUDRAGIT® RL).

Geeignet sind weiterhin auch z. B. neutrale (Meth)acrylat Copolymere aus 20 bis 40 Gew.-% Ethylacrylat und 60 bis 80 Gew.-% Methylmethacrylat (Typ EUDRAGIT® NE).

### Mischungen

Die bevorzugte Ausführungsform der Schicht b) sind Polymermischungen. Insbesondere die Polymertypen mit relative geringer Permeabilität EUDRAGIT® RS 30 D und EUDRAGIT® NE 30 D bewirken auch in geringen Schichtdicken eine therapeutisch unerwünscht starke Verzögerung der Wirkstoffabgabe. Aus diesem Grund verwendet man bevorzugt für die Schicht b) entweder den Polymertyp EUDRAGIT® RL mit höherer Permeabilität oder Mischungen aus EUDRAGIT® RL und EUDRAGIT® RS, z. B. im Verhältnis 9 zu 1 bis 1 zu 9. Geeignet ist insbesondere auch der Polymertyp EUDRAGIT® NE mit porenbildenden Zusätzen, wie z. B. NaCl, Saccharose, Hydroxypropylmethylcellulose (HPMC).

### Weitere Polymere

Zur Steuerung der Wirkstoffabgabe kann es im Einzelfall vorteilhaft sein, weitere Polymere zuzumischen. Der Anteil weiterer Polymere an der Mischung beträgt jedoch nicht mehr als 20 Gew.-%, bevorzugt höchstens 10 Gew.-%, insbesondere 0 - 5 Gew.-%, bezogen auf das (Meth)acrylat-Copolymere, beträgt.

Beispiele für solche weiteren Polymere sind: Polyvinylpyrolidon, Polyvinylalkohole, anionische (Meth)acrylat-Copolymere aus Methylmethacrylat und/oder Ethylacrylat und Methacrylsäure (EUDRAGIT® L 100, EUDRAGIT® S 100, EUDRAGIT® L 100-55). Anionische (Meth)acrylat-Copolymere aus Methylmethacrylat, Methylacrylat und Methacrylsäure, Carboxymethylcellulose-Salze, Hydroxypropylcellulose (HPMC), neutrale (Meth)acrylat Copolymere aus Methylmethacrylat und Ethylacrylat (Trockensubstanz aus EUDRAGIT® NE 30 D), Copolymere aus Methylmethacrylat und Butylmethacrylat (PLASTOID® B) oder (Meth)acrylat Copolymere mit quaternären Ammoniumgruppen (EUDRAGIT® RL bzw. EUDRAGIT® RS).

### Die Schicht b) enthält in der Regel weitere pharmazeutisch übliche Hilfsstoffe

### Äußere Schicht c)

Die äußere Schicht c) kann eine äußere magensaftresistente Umhüllung oder eine äußere Schicht mit einem magensaftresistenten Überzugsmittel sein. Sie hat die Aufgabe die verfrühte Freisetzung von Budesonid im Magen zu verhindern.

### Äußere magensaftresistente Umhüllung

Die äußere magensaftresistente Umhüllung kann eine Kapsel sein.
Die Kapsel besteht bevorzugt im wesentlichen aus Gelatine, oder aus Hydroxypropycellulose und insbesondere mit einem magensaftresistenten Überzug versehen sein.

Der magensaftresistente Überzug der Kapsel kann ein (Meth)acrylat-Copolymer sein, welches 40 bis 100 Gew.-% radikalisch polymerisierte Einheiten von C₁₋bis C₄-Alkylestern der Acryl- oder der Methacrylsäure und 5 bis zu 60 Gew.-% (Meth)acrylat-Monomeren mit einer anionischen Gruppe im Alkylrest enthält, Das (Meth)acrylat-Copolymer für den Überzug der Kapsel kann identisch oder verschieden sein von den Copolymeren der inneren und/oder der mittleren Schicht.

Die Kapseln enthalten den Wirkstoff in Form von Pellets oder Granulaten enthält. Die Pellets oder Granulate bestehen demnach aus der inneren wirkstoffhaltigen Schicht a) und der mittleren darmsaftlöslichen oder retardierenden Schicht b). Nach dem Auflösen der Kapsel in den oberen Darmabschnitten werden die enthaltenen Pellets oder Granulate freigesetzt.

### Äußere Schicht c) mit einem magensaftresistenten Überzugsmittel

Statt einer gefüllten Kapsel kann auch eine Formulierung, z. B. in Form von Pellets in Tablettenform vorliegen.

Das äußere magensaftresistente Überzugsmittel kann ein (Meth)acrylat-Copolymer ein, welches 40 bis 100 Gew.-% radikalisch polymerisierte Einheiten von C₁- bis C₄-Alkylestern der Acryl- oder der Methacrylsäure und 5 bis zu 60 Gew.-% (Meth)acrylat-Monomeren mit einer anionischen Gruppe im Alkylrest enthält.

Das (Meth)acrylat-Copolymer kann identisch oder verschieden sein von den Copolymeren der inneren und/oder der mittleren Schicht. Bevorzugt ist es verschieden vom (Meth)acrylat-Copolymer der mittleren Schicht.

Auch die Schicht c) enthält in der Regel weitere übliche pharmazeutische Hilfsstoffe.

### Kernmaterialien:

Die erfindungsgemäß optional eingesetzten Kerne sind wirkstofffreie Pellets oder Minitabletten im Korngrößenbereich zwischen 10 bis 3000 µm, bevorzugt 100 bis 1000 µm. Pellets bestehen bevorzugt aus Saccharose, Laktose oder Cellulose und werden durch Powder-Layering oder nach dem Feuchtextrusionsverfahren mit anschließender Ausrundung (Spheronisation) mit abschießender Trocknung hergestellt. Bevorzugt werden Saccharosepellets eingesetzt.

### Herstellung der Schichten b) und c):

Die Herstellung der Schichten b) und c) erfolgt mittels in der pharmazeutischen Verfahrenstechnik üblichen Verfahren, bevorzugt durch Sprühauftrag. Es ist jedoch auch ein Aufbringen der Schichten b) und c) durch Schmelzverarbeitung, wie auch bei der Schicht a), möglich. Man kann z. B. wirkstoffhaltige Folien erzeugen und Kerne darin einschweißen oder die Schicht durch Sprühauftrag im Schmelzezustand aufbringen.

### Ausführungsform in Anlehnung an WO 01/68058

Bevorzugt ist eine Ausführungsform in Anlehnung an WO 01/68058.
Dadurch kann eine Budesonid-Arzneiform bereitgestellt werden, die im Magen nahezu keinen Wirkstoff abgibt und eine gleichmäßige und langanhaltende Wirkstoffabgabe im Darm, insbesondere kurz vor oder erst im Dickdarmbereich ermöglicht. Die Art der Wirkstoffabgabe soll insbesondere die Anforderung erfüllen, daß im Freisetzungstest nach USP zwei Stunden bei pH 1,2 und anschließendem Umpuffern auf pH 7,0 der enthaltene Wirkstoff im Zeitraum bis 2,0 Stunden nach Testbeginn zu weniger als 5 % und zum Zeitpunkt acht Stunden nach Testbeginn zu 30 bis 80 % freisetzt wird.

Abweichend zu WO 01/68058 ist erfindungsgemäß die innere Schicht a) auf den Kern, die den Wirkstoff Budesonid, gebunden in einem polymeren Bindemittel mit sauren Gruppen enthält aufgebracht. Durch die auf diese Weise erreichte erhöhte Löslichkeit des Budesonids ergibt sich eine noch vorteilhaftere Ausführungsform.

### Mittlere Schicht b) gemäß WO 01/68058

Es folgt gemäß WO 01/68058 eine mittlere Schicht b) aus einem Copolymeren oder einer Mischung von Copolymeren, die sich aus 85 bis 98 Gew.-% radikalisch polymerisierten C1- bis C4-Alkylestern der Acryl- oder der Methacrylsäure und 15 bis 2 Gew.-% (Meth)acrylat-Monomeren mit einer quarternären Ammoniumgruppe im Alkylrest zusammensetzen.

Ein geeignetes Copolymer, kann z. B. aus aus 93 bis 98 Gew.-% radikalisch polymerisierten C1- bis C4-Alkylestern der Acryl- oder der Methacrylsäure und 7 - 2 Gew.-% 2-Trimethylammoniumethylmethacrylat-Chlorid hergestellt sein. Dabei können z. B. 50 - 70 Gew.-% Methylmethacrylat, 20 - 40 Gew.-% Ethylacrylat enthalten sein.

Ein entsprechendes Copolymer ist z. B. aus 65 Gew.-% Methylmethacrylat, 30 Gew.-% Ethylacrylat und 5 Gew.-% 2-Trimethylammoniumethylmethacrylat-Chlorid aufgebaut (EUDRAGIT® RS).

Ein weiteres geeignetes Copolymer, kann z. B. aus 85 bis weniger als 93 Gew.-% radikalisch polymerisierten C1- bis C4-Alkylestern der Acryl- oder der Methacrylsäure und mehr als 7 bis 15 Gew.-%
2-Trimethylammoniumethylmethacrylat-Chlorid hergestellt sein.

Geeignet ist ein Copolymer aus 60 Gew.-% Methylmethacrylat, 30 Gew.-% Ethylacrylat und 10 Gew.-% 2-Trimethylammoniumethlymethacrylat-Chlorid (EUDRAGIT® RL).

Der Mengenanteil der Schicht b) soll im Bereich von 2 bis 20 Gew.-% bezogen auf den Kern mit dem Wirkstoff betragen. Günstig ist die gleichzeitige Verwendung der beiden oben genannten Copolymer-Typen, bevorzugt derjenigen mit 5 und mit 10 Gew.-% 2-Trimethylammoniumethlymethacrylat-Chlorid (EUDRAGIT® RS und EUDRAGIT® RL) in Mischung. Das Mischungsverhältnis kann z. B. 20 : 1 bis 1 : 20, bevorzugt 10 : 1 bis 1 : 10 betragen

### Äußerer Schicht c) gemäß WO 01/68058

Es folgt eine äußere Schicht c) aus einem Copolymeren, das sich aus 75 bis 95 Gew.-% radikalisch polymerisierten C₁- bis C₄-Alkylestern der Acryl- oder der Methacrylsäure und 5 bis 60 Gew.-% (Meth)acrylat-Monomeren mit einer anionischen Gruppe im Alkylrest zusammensetzt, zur Herstellung einer Arzneiform, welche im Freisetzungstest nach USP zwei Stunden bei pH 1,2 und anschließendem Umpuffern auf pH 7,0 den enthaltenen Wirkstoff im Zeitraum bis 2,0 Stunden nach Testbeginn zu weniger als 5 % und zum Zeitpunkt acht Stunden nach Testbeginn zu 30 bis 80% freisetzt. Für die Therapie der Colitis Ulcerosa kann der äußere Überzug bevorzugt von Typ EUDRAGIT® FS sein.

Für die Therapie des Morbus Crohn, die auch schon in Abschnitten des Dünndarms auftritt kann der äußere Überzug bevorzugt von Typ EUDRAGIT® L sein. Geeignet sind ebenfalls anionische (Meth)acrylat Copolymere aus 20 bis 34 Gew.-% Methacrylsäure und/oder Acrylsäure, 20 bis 69 Gew.-% Methylacrylat und 0 bis 40 Gew.-% Ethylacrylat und gegebenenfalls 0 bis 10 Gew.-% weiteren vinylisch copolymerisierbarern Monomeren, mit der Maßgabe, daß die Glastemperatur des Copolymers nach ISO 11357-2, Punkt 3.3.3, höchstens 60 °C beträgt. (Typ EUDRAGIT® mit mittlerem Gehalt an Methacrylsäure).

Der Mengenanteil des äußeren Überzugs c) soll im Bereich von 10 bis 50 Gew.-% bezogen auf das Gewicht des Kerns mit dem Wirkstoff und dem inneren Überzug betragen.

### Pharmazeutisch übliche Hilfsstoffe

Die Schichten a), b) und c) können in an sich bekannter Weise weitere pharmazeutisch übliche Hilfsstoffe enthalten.

Bei der Herstellung der Arzneiform können pharmazeutisch übliche Hilfsstoffe in an sich bekannter Weise eingesetzt werden. Diese Hilfsstoffe können im Kern oder im Überzugsmittel enthalten sein.

### Trockenstellmittel (Antihaftmittel):

Trockenstellmittel haben folgende Eigenschaften: sie verfügen über große spezifische Oberflächen, sind chemisch inert, sind gut rieselfähig und feinteilig. Aufgrund dieser Eigenschaften erniedrigen sie die Klebrigkeit von Polymeren, die als funktionelle Gruppen polare Comonomere enthalten.

Beispiele für Trockenstellmittel sind:
Aluminiumoxid, Magnesiumoxid, Kaolin, Talkum, Kieselsäure (Aerosile), Bariumsulfat und Cellulose.

### Trennmittel

Beispiele für Trennmittel sind:
Ester von Fettsäuren oder Fettsäureamide , aliphatische, langkettige Carbonsäuren, Fettalkohole sowie deren Ester, Montan- oder Paraffinwachse und Metallseifen, insbesondere zu nennen sind Glycerolmonostearat, Stearylalkohol, Glycerolbehensäureester, Cetylalkohol, Palmitinsäure, Kanaubawachs, Bienenwachs etc.. Übliche Mengenanteile liegen im Bereich von 0,05 Gew-% bis 5, bevorzugt 0,1 bis 3 Gew.-% bezogen auf das Copolymere.

### Weitere pharmazeutisch übliche Hilfsstoffe:

Hier sind z. B, Stabilisatoren, Farbstoffe, Antioxidantien, Netzmittel, Pigmente, Glanzmittel etc. zu nennen. Sie dienen vor allem als Verarbeitungshilfsmittel und sollen ein sicheres und reproduzierbares Herstellungsverfahren sowie gute Langzeitlagerstabilität gewährleisten werden kann. Weitere pharmazeutisch übliche Hilfsstoffe können in Mengen von 0,001 Gew-% bis 30 Gew.-%, bevorzugt 0,1 bis 10 Gew.-% bezogen auf das Copolymere vorliegen.

### Weichmacher:

Als Weichmacher geeignete Stoffe haben in der Regel ein Molekulargewicht zwischen 100 und 20 000 und enthalten eine oder mehrere hydrophile Gruppen im Molekül, z. B. Hydroxyl- , Ester- oder Aminogruppen. Geeignet sind Citrate, Phthalate, Sebacate, Rizinusöl. Beispiele geeigneter Weichmacher sind Citronensäurealkylester, Propylenglykol, Glycerinester, Phthalsäurealkylester, Sebacinsäurealkylester, Sucroseester, Sorbitanester, Diethylsebacat , Dibutylsebacat und Polyethylenglykole 4000 bis 20.000. Bevorzugte Weichmacher sind Tributylcitrat, Triethylcitrat, Acetyltriethylcitrat, Dibutylsebacat und Diethylsebacat. Die Einsatzmengen liegen zwischen 1 und 60, bevorzugt 2 bis 20 Gew.-%, bezogen auf das filmbildende Polymer.

### Multipartikuläre Arzneiform

Eine weitere bevorzugt Ausführungsform ist durch die nachfolgend beschriebene multipartikuläre Arzneiform gegeben.

Die Multipartikuläre Arzneiform bewirkt eine vorteilhafte, weitgehend gleichmäßige Freisetzung von Budesonid im Dünndarm und im Dickdarm und enthält mindestens zwei verschiedene Pellettypen, wobei ein Pellettyp den Wirkstoff überwiegend im pH-Bereich des Dünndarm und der andere überwiegend im pH-Bereich des Dickdarm freisetzt.

Eine geeignete multipartikuläre Arzneiform kann z. B. zwei Formen von Pellets A und B enthalten. Auf einem Kern ist die innere Schicht a) mit dem gebundenen Budesonid enthalten, wobei die Pellet-Typen A und B zwei unterschiedliche Polymerüberzüge, mittlere Schichten b), aufweisen, die die Freisetzung des Wirkstoffs bei unterschiedlichen pH-Werten bestimmen.

Die die Pelletform A kann mit einem Polymerüberzug versehen sein, der eine kontinuierliche Wirkstofffreigabe ermöglicht, und einen äußeren magensaftresistenten Überzug aufweist, der sich oberhalb von etwa pH 5,5 schnell auflöst. Der äußere Überzug der Pelletform A kann z. B. EUDRAGIT® L100-55 sein.

Die Pelletform B mit kann mit einen Polymerüberzug, mittlere Schicht b), versehen sein, der im Freisetzungstest nach USP bei pH 6,8 in 6 Stunden weniger als 20 % des Wirkstoffs freisetzt und bei pH 7,2 in 6 Stunden mehr als 50 % des Wirkstoffs freisetzt.

Die Multipartikuläre Arzneiform kann in Form einer mit Pellets gefüllten Kapsel, z. B. einer Gelatinekapsel, vorliegen oder es kann sich um eine Tablette handeln, in der die Pellets zusammen mit üblichen Hilfsstoffen zur Tabletteneinheit verpreßt wurden.

Die Multipartikuläre Arzneiform ist geeignet zur weitgehend gleichmäßigen Freisetzung eines pharmazeutischen Wirkstoffs im Dünndarm und im Dickdarm und enthält mindestens zwei Formen von Pellets, A und B, die im Kern einen pharmazeutischen Wirkstoff enthalten, aber unterschiedliche Polymerüberzüge aufweisen, die die Freisetzung des Wirkstoffs bei unterschiedlichen pH-Werten bestimmen. In vitro erhält man im Freisetzungstest nach USP (USP 23, Methode 2) bei pH 6,8 und bei pH 7,2 Mischprofile die zwischen den individuellen Freisetzungskurven der beiden Pelletformen A und B liegen. In vivo dominiert im Dünndarm das Freisetzungsprofil der Pelletform A und während im Dickdarmbereich die Wirkstoffreigabe aus dem Pelletform B einsetzt.

Die Pelletkerne bestehen ganz oder teilweise aus einem pharmazeutischen Wirkstoff. Die Kerne sind in der Regel sphärisch oder rund und haben Durchmesser im Bereich von etwa 0,3 bis 2 mm. Die Polymerüberzüge liegen im Bereich von etwa 2 bis 16 mg Polymer pro cm² Oberfläche der Kerne.

### Pelletform A

Die Pelletform A ist mit einen inneren Polymerüberzug und einem äußeren Polymerüberzug versehen.

### Innerer Polymerüberzug

Der innere Polymerüberzug ermöglicht eine weitgehend pH-unabhängige kontinuierliche Wirkstofffreigabe. Es wird ein Wirkstofffreigabeprofil angestrebt, bei dem im Freisetzungstest nach USP (USP 23, Methode 2) bei pH 6,8 nach 2 Stunden etwa 40 bis 70 %, bevorzugt 40 bis 60 % und nach 4 Stunden 60 bis 100 %, bevorzugt 80 bis 100 % des Wirkstoffs freigesetzt werden. Dies leitet sich von der durchschnittlichen Verweildauer im Dünndarm, die etwa 4 Stunden beträgt ab.

Der innere Polymerüberzug der Pelletform A kann aus einem (Meth)acrylat-Copolymer, aus radikalisch polymerisierten C1- bis C4-Alkylestern der Acryl- oder der Methacrylsäure und (Meth)acrylat-Monomeren mit einer quaternären Ammoniumgruppe im Alkylrest bestehen.

Entsprechende (Meth)acrylat-Copolymere sind z. B. aus EP-A 181 515 oder aus DE-PS 1 617 751 bekannt. Es handelt sich um unabhängig vom pH-Wert lösliche oder quellbare Polymerisate, die für Arzneimittelüberzügen geeignet sind. Als mögliches Herstellungsverfahren ist die Substanzpolymeriation in Gegenwart eines im Monomerengemisch gelösten radikalbildenden Initiators zu nennen. Ebenso kann das Polymerisat auch mittels Lösungs- oder Fällungspolymerisation hergestellt werden. Das Polymerisat kann auf diese Weise in Form eines feinen Pulvers erhalten werden, was bei der Subtanzpolymerisation durch Mahlen, bei Lösungs- und Fällungspolymerisation z. B. durch Sprühtrocknung erreichbar ist.

Das (Meth)acrylat-Copolymer, setzt sich aus 85 bis 98 Gew.-% radikalisch polymerisierten C1- bis C4-Alkylestern der Acryl- oder der Methacrylsäure und 15 bis 2 Gew.-% (Meth)acrylat-Monomeren mit einer quaternären Ammoniumgruppe im Alkylrest zusammen.

Bevorzugte C1- bis C4-Alkylestern der Acryl- oder der Methacrylsäure sind Methylacrylat, Ethylacrylat, Butylacrylat, Butylmethacrylat und Methylmethacrylat.

Als (Meth)acrylat Monomer mit quaternären Ammoniumgruppen wird 2-Trimethylammoniumethylmethacrylat-Chlorid besonders bevorzugt.

Ein weiteres geeignetes (Meth)acrylat-Copolymer kann z. B. aus 85 bis weniger als 93 Gew.-% C1- bis C4-Alkylestern der Acryl- oder der Methacrylsäure und mehr als 7 bis 15 Gew.-% (Meth)acrylat Monomeren mit einer quaternären Ammoniumgruppe im Alkylrest aufgebaut sein. Derartige (Meth)acrylatMonomere sind handelsüblich und werden seit langem für retardierende Überzüge verwendet (Typ (EUDRAGIT® RL).

Ein konkret geeignetes Copolymer enthält z. B. 60 Gew.-% Methylmethacrylat, 30 Gew.-% Ethylacrylat und 10 Gew.-% 2-Trimethylammoniumethlymethacrylat-Chlorid (EUDRAGIT® RL).

Die gewünschte Freisetzungscharakteristik kann z. B. durch die Dicke der Überzugsschicht von Polymerüberzügen vom oben beschriebenen "Typ EUDRAGIT® RL" erreicht werden. Diese wird z. B. bei einem 5 bis 15 %-igem Überzug von EUDRAGIT® RL auf wirkstoffhaltigen Kernen mit 0,8 bis 1,2 mm Durchmesser erreicht. Die gewünschte Freisetzungscharakteristik kann auch bei anderen Schichtdicken durch Zumischen eines Copolymers, aus 50 - 70 Gew.-% Methylmethacrylat, 20 - 40 Gew.-% Ethylacrylat und 7 - 2 Gew.-% 2-Trimethylammoniumethylmethacrylat-Chlorid aufgebaut sein "(Typ EUDRAGIT® RS") erreicht werden. Ein konkret geeignetes Copolymer enthält 65 Gew.-% Methylmethacrylat, 30 Gew.-% Ethylacrylat und 5 Gew.-% 2-Trimethylammoniumethylmethacrylat-Chlorid aufgebaut sein (EUDRAGIT® RS). Die Typen EUDRAGIT® RL und RS können z. B. in den Verhältnissen 10 zu 1 bis 1 zu 10 gemischt werden. Bevorzugt sind höhere Anteile des "EUDRAGIT® RL-Typs", z. B. 60 bis 90 Gew.-% in der Mischung.

Der innere Polymerüberzug kann auch aus einem (Meth)acrylat Copolymeren aus 20 bis 40 Gew.-% Ethylacrylat und 60 bis 80 Gew.-% Methylmethacrylat, Ethylcellulose oder Polyvinylacetat bestehen.

### Äußerer Polymerüberzug

Der äußere Polymerüberzug ist ein magensaftresistenter Überzug, der sich erst oberhalb von etwa pH 5,5 schnell auflöst. Der Überzug soll somit eine Wirkstofffreisetzung im weitgehend Magen verhindern, d. h. diese soll nach USP 23 höchstens 10, bevorzugt nur 5 % betragen. Beim Übergang in den Dünndarm soll sich der äußere Polymerüberzug rasch auflösen, so daß die Freigabecharakteristik ab diesem Zeitpunkt vom inneren Polymerüberzug bestimmt wird.. Ist der äußere Polymerüberzug zu dünn, wird bereits im Magen zu viel Wirkstoff freigesetzt. Ist der äußere Polymerüberzug zu dick aufgetragen, behindert er die unmittelbare Wirkstofffreisetzung im Dünndarm. Geeignete Schichtdicken liegen z. B. im Bereich von 15 bis 150 µm, bevorzugt z. B. bei 20 bis 60 µm. Bezogen auf das Gewicht des mit dem inneren Polymerüberzug versehenen Kerns mit einem Durchmesser von 0,8 bis 1,25 mm, ist in der Regel ein Polymerauftrag (bezogen auf Trockensubstanz) im Bereich von 8 bis 40 Gew.-% , bevorzugt von 10 bis 25 Gew.-% geeignet.

Der magensaftresistente Polymerüberzug der Pelletform A kann aus einem säuregruppenhaltigen (Meth)acrylat-Copolymer, das z. B. Acrylsäure-, bevorzugt jedoch Methacrylsäure-Reste aufweist.

Das (Meth)acrylat-Copolymere besteht zu 40 bis 100, bevorzugt zu 45 bis 99, insbesondere zu 85 bis 95 Gew.-% aus radikalisch polymerisierten C₁- bis C₄₋Alkylestern der Acryl- oder der Methacrylsäure und kann 0 bis 60, bevorzugt 1 bis 55, insbesondere 5 bis 15 Gew.-% (Meth)acrylat-Monomere mit einer anionischen Gruppe im Alkylrest enthalten.

C₁- bis C₄-Alkylestern der Acryl- oder Methacrylsäure sind insbesondere Methylmethacrylat, Ethylmethacrylat, Butylmethacrylat, Methylacrylat, Ethylacrylat und Butylacrylat.

Geeignet sind z. B. auch neutrale (Meth)acrylat Copolymere aus 20 bis 40 Gew.-% Ethylacrylat und 60 bis 80 Gew.-% Methylmethacrylat (Typ EUDRAGIT® NE), wenn sie in Mischung mit säuregruppenhaltigen (Meth)acrylat-Copolymeren verwendet werden.

Besonders geeignet sind (Meth)acrylat Copolymere aus 40 bis 60, Gew.-% Methacrylsäure und 60 bis 40 Gew.-% Methylmethacrylat oder 60 bis 40 Gew.-% Ethylacrylat (Typen EUDRAGIT® L oder EUDRAGIT® L100-55).

Geeignet sind im Prinzip auch anionische (Meth)acrylat Copolymere aus 20 bis 40 Gew.-% Methacrylsäure und 80 bis 60 Gew.-% Methylmethacrylat (Typ EUDRAGIT® S).

Auch geeignet sind (Meth)acrylat Copolymere, bestehend aus 10 bis 30 Gew.-%, Methylmethacrylat, 50 bis 70 Gew.-% Methylacrylat und 5 bis 15 Gew.-% Methacrylsäure (Typ EUDRAGIT® FS).

Der magensaftresistente Polymerüberzug der Pelletform A kann auch aus Schellack, HPMCP (Hydroxypropylmethylcellulosephthalat), CAP (Celluloseacetatpththalat), HPMC-AS (Hydroxypropylmethylcellulose-Acetat-Succinat) oder Polyvinylacetatephthalat bestehen.

In jedem Fall ist jedoch darauf zu achten, daß der Überzug zum Beispiel in Bezug auf Schichtdicke und ggf. Mischung mit anderen Polymeren so eingestellt wird, daß er sich nach Übergang in den Dünndarm zügig auflöst.

### Pelletform B

Die Pelletform B setzt bei pH 6,8 im Freisetzungstest nach USP (USP 23, Methode 2) nach 2 Stunden nicht mehr als 10 %, bevorzugt nicht mehr als 5 % und nach 4 Stunden nicht mehr als 20, bevorzugt nicht mehr als 10 % des Wirkstoffs frei. Bei pH 7, 2 werden nach 3 Stunden etwa 40 bis 60 % und nach 60 Stunden etwa 80 bis 100 des Wirkstoffs freigesetzt.

Der Polymerüberzug für die Pelletform B kann ein (Meth)acyrlat-Copolymer sein, das sich aus 60 bis 95 Gew.-% radikalisch polymerisierten C₁- bis C₄₋Alkylestern der Acryl- oder der Methacrylsäure und 5 bis 40 Gew.-% (Meth)acrylat-Monomeren mit einer Säuregruppe im Alkylrest zusammensetzt.

Besonders gut geeignet sind (Meth)acrylat Copolymere, bestehend aus 10 bis 30 Gew.-%, Methylmethacrylat, 50 bis 70 Gew.-% Methylacrylat und 5 bis 15 Gew.-% Methacrylsäure (Typ EUDRAGIT® FS).

Ebenso geeignet sind (Meth)acrylat Copolymere aus 20 bis 40 Gew.-% Methacrylsäure und 80 bis 60 Gew.-% Methylmethacrylat (Typ EUDRAGIT® S).

### Verwendungen

Die erfindungsgemäße pharmazeutische Formulierung kann zur Therapie von Colitis Ulcerosa, Morbus Crohn und/oder weiteren, insbesondere entzündlichen Erkrankungen des Gastrointestinaltraktes, die mit Budesonid therapiert werden können, verwendet werden.

### Gehalt an Budesonid pro Dosiereinheit

Der Gehalt an Budesonid, bevorzugt mikronisiertem Budesonid, pro Dosiereinheit (Pellet) kann z. B. 0,5 bis 30 mg, bevorzugt 1 bis 10 mg betragen. Eine Dosiseinheit, eine Pellet-haltige Kapsel oder eine aus Pellets verpreßte Tablette kann z. B. 100 bis 1000, bevorzugt 150 bis 750 Pellets enthalten.

### BEISPIELE

Der Freigabetest für Budesonid erfolgt nach USP XXIII Monographie <711> "Dissolution" mit Apparat 2 (Paddle) bei einer Drehzahl von 100 / min in Phosphatpuffer pH 7,5 nach Monographie "Intestinal Fluid, Simulated, TS" ohne Zusatz von Pepsin oder in gereinigtem Wasser bei einer Drehzahl des Paddles von 100 / min mit 500 ml Lösungsmedium. Pro Bestimmung wurden 400 mg Probe eingewogen. Die Detektion erfolgte mittlels HPLC mit PP 18 Säule, 10 cm (Phenomenex) und UV Detektion bei 246 nm.
Geräte: Pumpe L 7000 100 (Fa. Merck-Hitachi, Darmstadt, Deutschland), Autosampler L 7000 200 (Fa. Merck-Hitachi, Darmstadt, Deutschland) UVNIS Detktor L 4250 ((Fa. Merck-Hitachi, Darmstadt, Deutschland).
Das Injektionsvolumen betrug 100µl, die Flussrate 1 ml/min. Die Retensionszeiten lagen durchschnittlich bei 2,5 min. Am Ende des Test wurden die Pellets mit Hilfe eines Ultra Turrax 10 min. homogenisiert. Der Gehalt ging als 100% Wert in die Berechnung ein. Es wurden 3 bis 6 Tests pro Medium durchgeführt.

### Beispiel 1 (nicht erfindunasaemäß): Bestimmung der Lösungsgeschwindigkeit von Budesonid ohne Bindemittel

Der Wirkstoff löst sich unter den angegebenen Bedingungen in vitro folgendermaßen auf:

| Zeit (min) | Freigabe in gereinigtem Wasser und Phosphatpuffer pH 7,5 (% der Theorie) | |
|---|---|---|
| | Mittelwert | Rel. Standardabweichung |
| 0 | 0,0 | 0,0 |
| 60 | ca.3,7 | 0,4 |
| 120 | ca. 5,4 | 1,0 |
| 180 | ca. 7,1 | 1,6 |

### Beispiel 2 (erfindungsgemäß): Einbettung von Budesonid in ein Bindemittel im Labormaßstab:

6 g Budesonid, 5 g Talkum und 1 g Triethylcitrat werden mit einem Homogemisator (Ultra Turrax, Fa. Jahnke & Kunkel, Deutschland) in 65 gereinigtem Wasser dispergiert und unter leichtem Rühren mit einem Propellerrüher mit 33 g EUDRAGIT® L 30 D-55 gemischt. Diese Sprühsuspension wird unter Rühren in einem Wirbelschichtgerät STREA 1 (Fa. Aeromatic, Bubendorf, Schweiz) auf 500 g Saccharose Pellets, 0,8. 1,0 mm (Fa. Werner, Tornesch, Deutschland) auf gesprüht.
Der Versuch wird durch folgende Daten beschrieben:

| | |
|---|---|
| Lacktrockensubstanz (LTS) [g] | 10 |
| Weichmacher bezogen auf LTS | 10% |
| Trennmittel bezogen auf LTS | 50% |
| Feststoffgehalt Dispersion (m/m) | 4,4% |
| LTS bezogen auf die Kernmasse | 2% |
| Überzugsgerät | Strea 1 |
| Pelletsorte | Sacch. |
| Düsendurchmesser [mm] | 0,8 |
| Sprühdruck [bar] | 0,5 |
| Ansatzgröße [g] | 500 |
| Auftragsmenge [g] | 110 |
| Vorwärmzeit [min] | 5 |
| Sprühzeit [min] | 52 |
| Zulufttemperatur [°C] | 41 |
| Ablufttemperatur [°C] | 30 |
| Sprührate [g/min] | 2,1 |
| Nachtrockenzeit [min] | 10 |

Die Pellets setzen den Wirkstoff unter den angegebenen Bedingungen in vitro wie unten beschrieben frei. Am Ende des Test wurden die Pellets mit Hilfe eines Ultra Turrax 10 min. homogenisiert und die Budesonid-Konzentration in der Lösung erneut bestimmt. Der letztere Meßwert ging als 100% Wert (theoretisch möglicher Budesonid-Gehalt in der Lösung) in die Berechnung ein.

| Zeit (min) | Freigabe in Phosphatpuffer pH 7,5 (% der Theorie) | | Freigabe in gereinigtem Wasser (% der Theorie) | |
|---|---|---|---|---|
| | Mittelwert | Rel. Standardabweichung | Mittelwert | Rel. Standardabweichung |
| 0 | 0,0 | 0,0 | 0,0 | 0,0 |
| 15 | 65,3 | 2,0 | 4,9 | 0,1 |
| 30 | 94,4 | 1,1 | 10,8 | 1,9 |
| 60 | 84,4 | 1,5 | 16,7 | 2,2 |
| 120 | 88,1 | 1,5 | 21,6 | 0,8 |

### Beispiel 3 (erfindungsgemäß): Einbettung von Budesonid in ein Bindemittel im Technikumsmaßstab:

36 g Budesonid, 60 g Talkum und 12 g Triethylcitrat werden mit einem Homogemisator (Ultra Turrax, Fa. Jahnke & Kunkel, Deutschland) in 632 gereinigtem Wasser dispergiert und unter leichtem Rühren mit einem Propellerrüher mit 33 g EUDRAGIT® L 30 D-55 gemischt. Diese Sprühsuspension wird unter Rühren in einem Wirbelschichtgerät WSG 5 (Fa. Glatt AG, Binzen, Deutschland) auf 6000 g Saccharose Pellets, 0,8.1,0 mm (Fa. Werner, Tornesch, Deutschland) auf gesprüht.

Der Versuch wird durch folgende Daten beschrieben:

| | |
|---|---|
| Lacktrockensubstanz (LTS) [g] | 120 |
| Weichmacher bezogen auf LTS | 10% |
| Trennmittel bezogen auf LTS | 50% |
| Feststoffgehalt Dispersion (m/m) | 3,8% |
| LTS bezogen auf die Kernmasse | 2% |
| Überzugsgerät | WSG 5 |
| Pelletsorte | Sacch. |
| Düsendurchmesser [mm] | 2,0 |
| Sprühdruck [bar] | 2,0 |
| Ansatzgröße [g] | 6000 |
| Auftragsmenge [g] | 1140 |
| Vorwärmzeit [min] | 5 |
| Sprühzeit [min] | 57 |
| Zulufttemperatur [°C] | 44 |
| Ablufttemperatur [°C] | 34 |
| Sprührate [g/min] | 20,0 |
| Nachtrockenzeit [min] | 10 |

Die Pellets setzen den Wirkstoff unter den angegebenen Bedingungen in vitro folgendermaßen frei:

| Zeit (min) | Freigabe (% der Theorie) | |
|---|---|---|
| | Mittelwert | Standardabweichung |
| 0 | 0,0 | 0,0 |
| 15 | 71,7 | 7,0 |
| 30 | 103,7 | 3,2 |
| 60 | 100, 5 | 4,3 |
| 120 | 98,3 | 5,1 |

### Beispiel 4: Weiterverarbeitung von Pelltes gemäß Beispiel 2 durch Aufbringen einer retardierenden Schicht b) und einer magensaftresitenten Schicht c) (Pharmazeutische Formulierung bzw. Arzneiform geeignet zur Therapie von Colitis Ulcerosa)

### Hersellung der Sprühsuspension 1 (Schicht b)):

8,75 g Talkum und 7 g Dibutylsebakat werden mit einem Homogemisator (Ultra Turrax, Fa. Jahnke & Kunkel, Deutschland) in 156,3 g gereinigtem Wasser dispergiert und unter leichtem Rühren mit einem Propellerrüher mit einer Mischung aus 26,3 g EUDRAGIT® RS 30 D und 8,8 g EUDRAGIT® RL 30 D gemischt.

350 g Pellets aus Beispiel 2 wurden in einem Wirbelschichtgerät STREA 1 (Fa. Aeromatic, Bubendorf, Schweiz) unter folgende Bedingungen überzogen:

| | |
|---|---|
| Lacktrockensubstanz (LTS) [g] | 35 |
| Weichmacher bezogen auf LTS | 20% |
| Trennmittel bezogen auf LTS | 50% |
| Feststoffgehalt Dispersion (m/m) | 17% |
| LTS bezogen auf die Kernmasse | 10% |
| Überzugsgerät | Strea 1 |
| Pelletsorte | Beisp. |
| | 2 |
| Düsendurchmesser [mm] | 0,8 |
| Sprühdruck [bar] | 0,5 |
| Ansatzgröße [g] | 350 |
| Auftragsmenge [g] | 297,5 |
| Vorwärmzeit [min] | 5 |
| Sprühzeit [min] | 117 |
| Zulufttemperatur [°C] | 40 |
| Ablufttemperatur [°C] | 329 |
| Sprührate [g/min] | 2,5 |
| Nachtrockenzeit [min] | 5 |

Die überzogenen Kerne werden anschließend auf Horden bei 40°C über 24 Std. im Umluftrockenschrank nachgetrocknet.

### Herstellung der Sprühsuspension 2 (Schicht c)):

1,3 g Glycerolmonostearat, 1,3 g Triethylcitrat und 0,5 g Polysorbat 80 werden mit einem Homogemisator (Ultra Turrax, Fa. Jahnke & Kunkel, Deutschland) in 156,3 g gereinigtem Wasser dispergiert und unter leichtem Rühren mit einem Propellerrüher mit 87,5 g EUDRAGIT® FS 30 D gemischt.

400 g Pellets aus Beispiel 2 mit Retardüberzug aus Sprühsuspension 1 wurden in einem Wirbelschichtgerät STREA 1 (Fa. Aeromatic, Bubendorf, Schweiz) unter folgende Bedingungen überzogen:

| | |
|---|---|
| Lacktrockensubstanz (LTS) [g] | 30 |
| Weichmacher bezogen auf LTS | 3% |
| Trennmittel bezogen auf LTS | 5% |
| Feststoffgehalt Dispersion (m/m) | 20% |
| LTS bezogen auf die Kernmasse | 7,5% |
| Überzugsgerät | Strea 1 |
| Pelletsorte | Beisp. |
| | 2 |
| Düsendurchmesser [mm] | 0,8 |
| Sprühdruck [bar] | 0,5 |
| Ansatzgröße [g] | 400 |
| Auftragsmenge [g] | 165 |
| Vorwärmzeit [min] | 5 |
| Sprühzeit [min] | 54 |
| Zulufttemperatur [°C] | 31 |
| Ablufttemperatur [°C] | 25 |
| Sprührate [g/min] | 3,1 |
| Nachtrockenzeit [min] | 5 |

Die überzogenen Kerne werden anschließend auf Horden bei 40°C über 2 Std. im Umluftrockenschrank nachgetrocknet.

Die mit den Schichten b) und c) überzogenen Pellets setzen der Wirkstoff unter den angegebenen Bedingungen in vitro folgendermaßen frei:

| Zeit (min) | Freigabe in Phosphatpuffer pH 7,5 (% der Theorie) | |
|---|---|---|
| | Mittelwert | Rel. Standardabweichung |
| 0 | 0,0 | 0 |
| 30 | 0,0 | 0 |
| 60 | 0,0 | 0 |
| 120 | 1,8 | 0,1 |
| 180 | 4,2 | 0,3 |
| 240 | 11,7 | 1,8 |
| 300 | 32,6 | 2,0 |
| 360 | 50,5 | 7,4 |
| 480 | 73,3 | 7,0 |
| 600 | 85,3 | 3,0 |

## Patentansprüche

1. Pharmazeutische Formulierung, enthaltend im wesentlichen
a) eine innere Schicht, die gegebenenfalls auf einen Kern aufgetragen sein kann, mit dem Wirkstoff Budesonid, gebunden in einem Bindemittel
b) eine mittlere Schicht mit einem darmsaftlöslichen oder retardierenden, polymeren Überzugsmittel,
c) eine äußere magensaftresistente Umhüllung oder eine äußere Schicht mit einem magensaftresistenten Überzugsmittel
wobei die Schichten in an sich bekannter Weise weitere pharmazeutisch übliche Hilfsstoffe enthalten können,
**dadurch gekennzeichnet, daß**
das Bindemittel ein Polymer oder Copolymer mit sauren Gruppen ist und die Formulierung der inneren Schicht ohne mittlere und äußere Schicht den gebundenen Wirkstoff im Freisetzungsversuch nach USP XXIII Monographie <711> "Dissolution" mit Apparat 2 (Paddle) bei einer Drehzahl von 100 / min in Phosphatpuffer pH 7,5 nach 30 min zu mehr als 80 % freisetzt.

2. Pharmazeutische Formulierung nach Anspruch 1, **dadurch gekennzeichnet, daß** das polymere Bindemittel ein (Meth)acrylat-Copolymer ist, welches 40 bis 95 Gew.-% radikalisch polymerisierte Einheiten von C₁- bis C₄-Alkylestern der Acryl- oder der Methacrylsäure und 5 bis 60 Gew.-% (Meth)acrylat-Monomeren mit einer anionischen Gruppe im Alkylrest enthält.

3. Pharmazeutische Formulierung nach Anspruch 1 oder 2, **dadurch gekennzeichnet, daß** das polymere Bindemittel ein Vinylpyrrolidon-Vinylacetat-Copolymer ist.

4. Pharmazeutische Formulierung nach einem oder mehreren der Ansprüche 1 bis 3, **dadurch gekennzeichnet, daß** die mittlere Schicht ein ein (Meth)acrylat-Copolymer ist, welches 40 bis 100 Gew.-% radikalisch polymerisierte Einheiten von C₁- bis C₄-Alkylestern der Acryl- oder der Methacrylsäure und keine oder bis zu 60 Gew.-% (Meth)acrylatMonomeren mit einer anionischen Gruppe im Alkylrest enthält.

5. Pharmazeutische Formulierung nach einem oder mehreren der Ansprüche 1 bis 3, **dadurch gekennzeichnet, daß** die mittlere Schicht ein (Meth)acrylat-Copolymer ist, welches 85 bis 98 Gew.-% radikalisch polymerisierten Einheiten von C1- bis C4-Alkylester der Acryl- oder der Methacrylsäure und 15 bis 2 Gew.-% (Meth)acrylat-Monomeren mit einer quaternären Ammoniumgruppe im Alkylrest enthält.

6. Pharmazeutische Formulierung nach einem oder mehreren der Ansprüche 1 bis 5, **dadurch gekennzeichnet, daß** das äußere magensaftresistente Überzugsmittel ein (Meth)acrylat-Copolymer ist, welches 40 bis 100 Gew.-% radikalisch polymerisierte Einheiten von C₁₋bis C₄-Alkylestern der Acryl- oder der Methacrylsäure und 5 bis zu 60 Gew.-% (Meth)acrylat-Monomeren mit einer anionischen Gruppe im Alkylrest enthält.

7. Pharmazeutische Formulierung nach einem oder mehreren der Ansprüche 1 bis 5, **dadurch gekennzeichnet, daß** die äußere magensaftresistente Umhüllung eine Kapsel ist.

8. Pharmazeutische Formulierung nach Anspruch 6, **dadurch gekennzeichnet, daß** die Kapsel im wesentlichen aus Gelatine oder aus Hydroxypropycellulose besteht.

9. Pharmazeutische Formulierung nach Anspruch 6 oder 7, **dadurch gekennzeichnet, daß** die Kapsel mit einem magensaftresistenten Überzug versehen ist.

10. Pharmazeutische Formulierung nach Anspruch 6, 7 oder 8, **dadurch gekennzeichnet, daß** sie den Wirkstoff in Form von Pellets oder Granulaten enthält.

11. Pharmazeutische Formulierung nach einem oder mehreren der Ansprüche 1 bis 10, **dadurch gekennzeichnet, daß** es sich um eine multipartikuläre Arzneiform mit weitgehend gleichmäßigen Freisetzung von Budesonid im Dünndarm und im Dickdarm handelt, die mindestens zwei verschiedene Pellettypen enthält, wobei ein Pellettyp den Wirkstoff überwiegend im pH-Bereich des Dünndarm und der andere überwiegend im pH-Bereich des Dickdarm freisetzt.

12. Pharmazeutische Formulierung nach Anspruch 11, **dadurch gekennzeichnet, daß** die Pellets in einer Kapsel nach einem oder mehreren der Ansprüche 6 bis 10 eingeschlossen sind.

13. Pharmazeutische Formulierung nach Anspruch 11, **dadurch gekennzeichnet, daß** daß die Pellets in Form einer Tablette vorliegen, in der die Pellets zusammen mit üblichen Hilfsstoffen zur Tabletteneinheit verpreßt wurden.

14. Verfahren zur Herstellung einer pharmazeutische Formulierung nach einem oder mehreren der Ansprüche 1 bis13, **dadurch gekennzeichnet, daß** man zunächst eine innere Schicht a) in der Budesonid in einem polymeren Bindemittel mit sauren Gruppen gebunden ist, in an sich bekannter,Weise durch Sprühauftrag oder Schmelzverarbeitung erzeugt, wobei die innere Schicht a) gegebenenfalls auf einen Kern aufgetragen wird, und anschließend die mittlere Schicht b) und die äußere Schicht c) in an sich bekannter Weise durch Sprühauftrag oder Schmelzverarbeitung aufbringt.

15. Verfahren zur Herstellung einer pharmazeutische Formulierung nach Anspruch 14, **dadurch gekennzeichnet, daß** man ein Bindemittel gemäß Anspruch 2 in Form einer Dispersion einsetzt und die innere Schicht a) durch wäßriges Sprühen einer Budesonid-haltigen (Meth)acrylat-Copolymer-Dispersion auf Kerne unter Bindung des Budesonids nach dem Abdampfen des Wassers erzeugt.

16. Verwendung einer pharmazeutische Formulierung nach einem oder mehreren der Ansprüche 1 bis 13 als Arzneiform zur Therapie von Colitis Ulcerosa, Morbus Crohn und/oder weiteren Erkrankungen des Gastrointestinaltraktes, die mit Budesonid therapiert werden können.

## Claims

1. Pharmaceutical formulations essentially containing
a) an inner layer which may optionally be applied to a core, containing the active substance budesonide, bound in a binder
b) a middle layer having a polymeric coating which is soluble in intestinal juices or which delays release,
c) an outer shell resistant to gastric juices or an outer layer having a coating resistant to gastric juices,
while the layers may contain other pharmaceutical conventional excipients in a manner known *per se,*
**characterised in that**
the binder is a polymer or copolymer with acid groups and the formulation of the inner layer without the middle and outer layers releases more than 80% of the bound active substance after 30 minutes in the release test according to USP XXIII Monograph <711> "Dissolution" with apparatus 2 (paddle) at a rotation speed of 100/min in phosphate buffer pH 7.5.

2. Pharmaceutical formulation according to claim 1, **characterised in that** the polymeric binder is a (meth)acrylate copolymer which contains 40 to 95 wt.% of radically polymerised units of C₁-C₄ alkyl esters of acrylic or methacrylic acid and 5 to 60 wt.% of (meth)acrylate monomers with an anionic group in the alkyl moiety.

3. Pharmaceutical formulation according to claim 1 or 2, **characterised in that** the polymeric binder is a vinylpyrrolidone-vinylacetate copolymer.

4. Pharmaceutical formulation according to one or more of claims 1 to 3, **characterised in that** the middle layer is a (meth)acrylate copolymer which contains 40 to 100 wt.% of radically polymerised units of C₁-C₄ alkyl esters of acrylic or methacrylic acid and no or up to 60 wt.% of (meth)acrylate monomers having an anionic group in the alkyl moiety.

5. Pharmaceutical formulation according to one or more of claims 1 to 3, **characterised in that** the middle layer is a (meth)acrylate copolymer which contains 85 to 98 wt.% of radically polymerised units of C₁-C₄ alkyl esters of acrylic or methacrylic acid and 15 to 2 wt.% of (meth)acrylate monomers with a quaternary ammonium group in the alkyl moiety.

6. Pharmaceutical formulation according to one or more of claims 1 to 5, **characterised in that** the outer gastric juice-resistant coating is a (meth)acrylate copolymer which contains 40 to 100 wt.% of radically polymerised units of C₁-C₄ alkyl esters of acrylic or methacrylic acid and 5 to 60 wt.% of (meth)acrylate monomers with an anionic group in the alkyl moiety.

7. Pharmaceutical formulation according to one or more of claims 1 to 5, **characterised in that** the outer shell resistant to gastric juices is a capsule.

8. Pharmaceutical formulation according to claim 6, **characterised in that** the capsule consists essentially of gelatine or hydroxypropylcellulose.

9. Pharmaceutical formulation according to claim 6 or 7, **characterised in that** the capsule is provided with a gastric juice-resistant coating.

10. Pharmaceutical formulation according to claim 6, 7 or 8, **characterised in that** it contains the active substance in the form of pellets or granules.

11. Pharmaceutical formulation according to one or more of claims 1 to 10, **characterised in that** it is a multiparticulate medicament with a substantially uniform release of budesonide in the small intestine and in the large intestine, which contains at least two different types of pellet, one type of pellet releasing the active substance predominantly in the pH range of the small intestine while the other type of pellet releases it predominantly in the pH range of the large intestine.

12. Pharmaceutical formulation according to claim 11, **characterised in that** the pellets are enclosed in a capsule according to one or more of claims 6 to 10.

13. Pharmaceutical formulation according to claim 11, **characterised in that** the pellets are in the form of a tablet in which the pellets have been compressed together with conventional excipients to form the tablet unit.

14. Method of producing a pharmaceutical formulation according to one or more of claims 1 to 13, **characterised in that** first of all an inner layer a) in which budesonide is bound in a polymeric binder with acid groups is produced in a manner known *per se* by spray application or processing in a melt, while the inner layer a) is optionally applied to a core, and then the middle layer b) and the outer layer c) are applied in a manner known *per se* by spray application or processing in a melt.

15. Process for producing a pharmaceutical formulation according to claim 14, **characterised in that** a binder according to claim 2 is used in the form of a dispersion and the inner layer a) is produced by aqueous spraying of a budesonide-containing (meth)acrylate copolymer dispersion on to cores, the budesonide being bound after the water evaporates.

16. Use of a pharmaceutical formulation according to one or more of claims 1 to 13 as a medicament for the treatment of ulcerative colitis, Crohn's disease and/or other diseases of the gastrointestinal tract which can be treated with budesonide.

## Revendications

1. Formulation pharmaceutique contenant pour l'essentiel
a) une couche interne qui peut le cas échéant être déposée sur un noyau, avec le principe actif budesonide, fixé dans un liant,
b) une couche médiane avec un revêtement polymère soluble dans le suc intestinal ou retardant,
c) un enrobage externe résistant au suc gastrique ou une couche externe avec un revêtement résistant au suc gastrique,
les couches pouvant contenir de façon connue d'autres adjuvants pharmaceutiques habituels,
**caractérisée en ce que**
le liant est un polymère ou copolymère avec des groupes acides, et la formulation de la couche interne sans couche médiane et sans couche externe libère à plus de 80 % le principe actif lié, dans l'expérience de libération selon la monographie de la pharmacopée américaine USP XXIII <711> « Dissolution » avec l'appareil 2 (palette), à une rotation de 100 tours par minute, dans un tampon phosphaté à pH 5, au bout de 30 minutes.

2. Formulation pharmaceutique selon la revendication 1,
**caractérisée en ce que**
le liant polymère est un copolymère de (méth)acrylate qui contient de 40 à 95 % en poids d'unités à polymérisation radicalaire d'esters alkyliques en C₁ à C₄ de l'acide acrylique ou de l'acide méthacrylique, et de 5 à 60 % en poids de monomères de (méth)acrylate avec un groupe anionique dans le radical alkyle.

3. Formulation pharmaceutique selon la revendication 1 ou 2,
**caractérisée en ce que**
le liant polymère est un copolymère vinylpyrrolidone-acétate de vinyle.

4. Formulation pharmaceutique selon une ou plusieurs des revendications 1 à 3,
**caractérisée en ce que**
la couche médiane est un copolymère de (méth)acrylate qui contient de 40 à 100 % en poids d'unités à polymérisation radicalaire d'esters alkyliques en C₁ à C₄ de l'acide acrylique ou de l'acide méthacrylique, et aucun ou jusqu'à 60 % en poids de monomères de (méth)acrylate avec un groupe anionique dans le radical alkyle.

5. Formulation pharmaceutique selon une ou plusieurs des revendications 1 à 3,
**caractérisée en ce que**
la couche médiane est un copolymère de (méth)acrylate qui contient de 85 à 98 % d'unités à polymérisation radicalaire d'esters alkyliques en C₁ à C₄ de l'acide acrylique ou de l'acide méthacrylique, et de 15 à 2 % en poids de monomères de (méth)acrylate avec un groupe ammonium quaternaire dans le radical alkyle.

6. Formulation pharmaceutique selon une ou plusieurs des revendications 1 à 5,
**caractérisée en ce que**
le revêtement externe résistant au suc gastrique est un copolymère de (méth)acrylate qui contient de 40 à 100 % en poids d'unités à polymérisation radicalaire d'esters alkyliques en C₁ à C₄ de l'acide acrylique ou de l'acide méthacrylique, et de 5 à 60 % en poids de monomères de (méth)acrylate avec un groupe anionique dans le radical alkyle.

7. Formulation pharmaceutique selon une ou plusieurs des revendications 1 à 5,
**caractérisée en ce que**
l'enrobage externe résistant au suc gastrique est une capsule.

8. Formulation pharmaceutique selon la revendication 6,
**caractérisée en ce que**
la capsule se compose essentiellement de gélatine ou d'hydroxypropylcellulose.

9. Formulation pharmaceutique selon la revendication 6 ou 7,
**caractérisée en ce que**
la capsule est munie d'un revêtement résistant au suc gastrique.

10. Formulation pharmaceutique selon l'une des revendications 6, 7 ou 8,
**caractérisée en ce qu'**
elle contient le principe actif sous forme de pastilles ou de granulés.

11. Formulation pharmaceutique selon une ou plusieurs des revendications 1 à 10,
**caractérisée en ce qu'**
il s'agit d'une forme médicamenteuse multiparticulaire avec libération largement régulière de budesonide dans l'intestin grêle et dans le gros intestin, et qui contient au moins deux types de pastilles différents, un type de pastille libérant le principe actif principalement dans l'intervalle de pH de l'intestin grêle et l'autre principalement dans l'intervalle de pH du gros intestin.

12. Formulation pharmaceutique selon la revendication 11,
**caractérisée en ce que**
les pastilles sont incluses dans une capsule selon une ou plusieurs des revendications 6 à 10.

13. Formulation pharmaceutique selon la revendication 11,
**caractérisée en ce que**
les pastilles se présentent sous la forme d'un comprimé dans lequel les pastilles sont pressées ensemble avec des adjuvants habituels pour fournir le contenu du comprimé.

14. Procédé de fabrication d'une formulation pharmaceutique selon une ou plusieurs des revendications 1 à 13,
**caractérisé en ce qu'**
on produit tout d'abord une couche interne a) dans laquelle le budesonide est fixé dans un liant polymère avec des groupes acides, en procédant de façon connue au moyen d'un dépôt par pulvérisation ou d'un traitement à l'état fondu, la couche interne a) étant le cas échéant déposée sur un noyau, puis
on introduit de façon connue la couche médiane b) et la couche externe c) au moyen d'un dépôt par pulvérisation ou d'un traitement à l'état fondu.

15. Procédé de fabrication d'une formulation pharmaceutique selon la revendication 14,
**caractérisé en ce qu'**
on utilise un liant selon la revendication 2 sous la forme d'une dispersion et
on produit la couche interne a) au moyen d'une pulvérisation aqueuse d'une dispersion de copolymère de (méth)acrylate contenant du budesonide sur des noyaux, avec fixation du budesonide après évaporation de l'eau.

16. Utilisation d'une formulation pharmaceutique selon une ou plusieurs des revendications 1 à 13, comme forme médicamenteuse pour le traitement de la colite ulcéreuse, de la maladie de Crohn et/ou d'autres maladies de l'appareil gastro-intestinal qui peuvent être traitées avec le budesonide.
